(19) Europäisches Patentamt European Patent Office Office européen des brevets

(11) **EP 3 859 745 A1**

(12) **EUROPEAN PATENT APPLICATION**

(43) Date of publication:
**04.08.2021 Bulletin 2021/31**

(51) Int Cl.:
**G16H 50/50** (2018.01)   **G16H 70/40** (2018.01)

(21) Application number: **20386008.5**

(22) Date of filing: **03.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **National Centre for Scientific Research "Demokritos"**
**15341 Agia Paraskevi (GR)**

(72) Inventors:
• **Bougatiotis, Konstantinos**
**15310 Aghia Paraskevi (GR)**
• **Aisopos, Fotis**
**15310 Aghia Paraskevi (GR)**
• **Nentidis, Anastasios**
**15310 Aghia Paraskevi (GR)**
• **Paliouras, Georgios**
**15310 Aghia Paraskevi (GR)**

(74) Representative: **Käck, Stefan**
**Kahler Käck Mollekopf**
**Partnerschaft von Patentanwälten mbB**
**Vorderer Anger 239**
**86899 Landsberg/Lech (DE)**

Remarks:
Amended claims in accordance with Rule 137(2) EPC.

(54) **SYSTEM AND METHOD FOR IDENTIFYING DRUG-DRUG INTERACTIONS**

(57)    Methods and systems for predicting possible interactions between two or more drugs are disclosed. A plurality of documents is analyzed (212, 214) to extract medical relevant information. The extracted information comprises at least one first entity associated with at least one second entity. In addition, supplementary information is received (220) from a structured data source (220-1, 220-2). A graph is then created to represent the information extracted from the plurality of documents and the supplementary information from the structured data source (220-1, 220-2) in a unified scheme. The created graph comprises a plurality of nodes and a plurality of edges connecting the plurality of nodes. After the creation of the graph, the graph is analyzed (234) using machine learning techniques to identify at least one new edge. The identification of the at least one new edge is based on paths already included in the initially created graph. Based on the at least one new edge, probable drug-to-drug interactions are determined (236) and output.

Fig. 2

EP 3 859 745 A1

## Description

## TECHNICAL FIELD

The present invention relates to the identification of drug-drug interactions. More particular, it relates to an automated system and method for estimating the likelihood that two or more drugs interact with each other when administered together.

## BACKGROUND

A change in the action or side effect of a drug caused by concomitant administration with other drugs is referred to as drug-drug interaction (DDI) or multidrug interaction. A drug-drug interaction can delay, decrease, or enhance absorption of either drug. This can decrease or increase the action of either or both drugs or cause adverse effects. Drug-drug interactions can therefore pose a serious problem for patient safety, cause severe injuries, or even lead to death. As published by Percha et al. in an article entitled "Informatics confronts drug-drug interactions", the extend of the problem becomes more evident given that, in the United States alone for example, drug-drug interactions are responsible for up to 195,000 hospital admissions. Therefore, identifying drugs interacting with each other is a crucial need for clinicians, in order to be able to predict and avoid unnecessary implications.

Various tools for finding and identifying potentially harmful and unsafe combinations of prescription medications already exist. For example, the websites drugs.com and webmd.com provide online drug interaction checkers. These checkers allow finding drug pairs that are known to interact with each other.

Drug-drug interactions are however difficult to detect in the different stages of drug development and in post-marketing surveillance. Current processes of drug-drug interaction prediction are highly technical and domain expertise from human experts is needed. Determining whether a certain drug interacts with another drug therefore is an expensive and time-consuming task. Clinical trials, elaborate laboratory tests or medical experiments on animals may be necessary to identify interacting drug pairs. In addition, the number of different drugs makes it nearly impossible to test all possible combinations of drugs.

One exemplary method for predicting the likelihood of an interaction between two different drugs is disclosed in U.S. Patent No. 6,370,478. In the described approach, a plurality of cellular constituents in a biological sample is monitored as the sample is subject to various drug treatments. The responses of the cellular constituents are then analyzed to detect interactions.

There is accordingly a need to mitigate at least some of the drawbacks mentioned above. In particular, there is a need to identify or predict an interaction of two different drugs by a fast, reliable and resource-saving process, which avoids or at least reduces the necessity of clinical trials, laboratory tests and/or medical experiments on animals.

## SUMMARY

The invention is defined in the appended independent claims. Particular embodiments are set out in the dependent claims.

A computer-implemented method for estimating a likelihood that two or more drugs interact with each other is provided. A plurality of documents is analyzed to extract information. The extracted information comprises at least one first entity associated with at least one second entity. In addition, supplementary information is received from a structured data source. The method then creates a graph to represent the information extracted from the plurality of documents and the supplementary information from the structured data source in a unified scheme. The created graph comprises a plurality of nodes and a plurality of edges connecting the plurality of nodes. After the creation of the graph, the graph is analyzed using machine learning techniques to identify at least one new edge. The identification of the at least one new edge is based on paths already included in the initially created graph. Based on the at least one new edge, probable or possible drug-to-drug interactions are determined as output.

In particular, the method identifies and/or predicts probable interactions between two or more drugs in an automated manner.

The expression "drug", as used herein, refers to any substance that causes a change in an organism's physiology or psychology when consumed. Drugs may be chemical substances used to treat, cure, prevent, or diagnose a disease or to promote well-being. For example, the interaction between two pharmaceutical drugs, also called medications or medicines, may be examined and estimated by the method. Drugs may be a single substance or a compound of two or more substances.

The effect of or the change in the physiology or psychology of a living being (e.g., a patient) when a first drug is taken together with a second drug is referred to as a drug-drug interaction. The drug-drug interaction may be desired or positive for the patient (e.g., the concomitant administration of two drugs speeds up a healing process) or the drug-drug interaction may be undesired or negative (e.g., two drugs in combination pose a serious health risk for a patient). Alternatively, the coadministration of two or more drugs at the same time does not have any effect in addition to those these two or more drugs already have when taken alone, which means that in this case the two or more drugs do not interact with each other.

The aim of the present invention is to determine or estimate the likelihood whether two or more drugs, when administered together, have an additional effect (e.g., pose a health risk), without prior knowledge that or whether the two or more drugs interact.

In order to achieve this, the method performs an analysis of the plurality of documents to extract (bio)medical and/or (bio)chemical relevant information. The extracted information comprises at least one first entity associated with at least one second entity.

The plurality of documents contains medical and/or biomedical information such as information about drugs, disease, proteins, known interacting drug pairs, etc.. The documents may comprise scientific publications, articles in journals, and/or biomedical literature. It is preferred that complete documents (i.e., full-text) are used as basis for the analysis so that all aspects included in the documents are taken into account. Alternatively or additionally, some or all documents may comprise only portions or abstracts of complete documents.

Certain information is extracted from the documents. For example, the documents may explain that two drugs interact with each other and that the coadministration of these two drugs poses a health risk for patients. Alternatively or additionally, the documents may describe certain causal relationships between two entities. The documents therefore represent an initial collection of known associations or relations between entities.

Different techniques for automatically extracting the information from the plurality of documents may be used. The different extraction techniques may be used alone or in combination. For example, biomedical entities and relations between them may be extracted from the plurality of documents by a data mining process.

Instead of performing an analysis of the plurality of documents to extract associations between entities, the method may also use an already existing collection of associated entity pairs. The already existing collection of associated entity pairs may be the result of previous extraction and analysis processes or may be provided by a third party.

In addition to the information extraction and analysis of the plurality of documents, the method also receives supplementary information from one or more data sources of structured data.

In particular, the method may take additional knowledge stored in existing, high-quality repositories into account to complement the information extracted from the plurality of documents and to improve the prediction. The structured data source may comprise knowledge bases, ontologies, and/or manually-curated databases. The information in the structured data source may be collected or provided by experts in the respective fields, by a certain organization, or is maintained by a plurality of volunteers. For example, biomedical ontologies available in the Open Biomedical and Biological Ontologies may be used to enrich and analyze the information extracted from the plurality of documents.

Based on the information extracted from the plurality of documents and the supplementary information from the structured data source, the method then creates or generates a graph to store and process all the information in a unified semantic scheme. A graph is a well-known data structure which comprises nodes (also called vertices) and edges between the nodes (also called links or lines).

The method thus allows incorporating information from multiple data sources and of different data types or formats (e.g., structured and un-structured data sources) into a common scheme, using the same vocabulary.

The created graph may be stored in a graph database to more efficiently analyze and process it. Graph databases are databases that use graph structures for semantic queries with nodes, edges, and properties to represent and store data. One example for a graph database management system is Neo4j developed by Neo4j, Inc. Alternatively or additionally, the Virtuoso Universal Server developed by OpenLink Software, Inc. may be used to store and/or process the graph.

After the graph is created, the graph is analyzed using machine learning techniques to identify at least one new edge. The analysis of the graph and the identification of the at least one new edge are based on paths included in the initially created graph.

In particular, new edges between nodes may be determined or predicted based on the combination of existing graph edges or paths. The new edges are not included in the initially created graph. In other words, the at least one new edge is not included in or extractable from the plurality of documents and is also not part of the supplementary information stored in the structured data source.

The determination of new edges may be formulated as a graph completion problem (or link prediction problem). For a set of drugs D and relevant paths P existing in the graph, as well as an initial collection of known interacting drug pairs $I = \{(d_i, d_j) | d_i, d_j \in D \text{ and } d_i \leftrightarrow d_j\}$ where $\leftrightarrow$ denotes a drug-drug-interaction, the analysis of the graph therefore has the goal to identify unknown pairs $(d_i', d_j') \notin I$ of interacting drugs.

The method thus uses paths existing in the initially created graph (including facts and information derived from the plurality of documents and the structured data sources) to infer new edges (or at least the probability of their existence) so as to build a more complete graph.

The analysis of the graph uses machine learning techniques. For example, the paths between examined drug pairs may be used as training data for a classifier (e.g., Random Forest).

The method then determines possible drug-drug interactions based on the at least one new edge. This can be done by providing to the trained classifier a set of drug pairs that there is no previously known interaction between them. The classifier responds with a probability (confidence) score of an interaction between each one of those pairs, which is translated as the existence of relevant edges in the graph between respective nodes.

The possible drug-drug interaction may also be output. For example, the results may be displayed to a user via a graphical user interface. The potential interactions between the different drugs may also be manually invested or examined in laboratory tests.

As the method is fully automatized, it may be integrated into existing software applications for clinicians or medical personnel to consistently monitor administered drugs based on electronic patient records and to warn clinicians or medical personnel if potentially harmful drugs are administered together. For example, the two drugs to be examined may be automatically extractable from the electronic patient record. In such a case, the determination may be implemented as a background process which does not need to be explicitly started by an end user.

The method thus allows predicting possible drug-drug interactions by an automated decision-making software solution which can warn clinicians or medical personnel about potentially dangerous drug combinations. As the underlying data for the identification and predication is extracted from existing documents, the method avoids or at least reduces new biochemical or biomedical analyses and laboratory tests. For that reason, the prediction of drug-drug interactions as disclosed herein provides a fast and resource-saving alternative to current approaches for finding and identifying potentially harmful and unsafe combinations of prescription medications.

The results of the method may also be used in combination with traditional tests or experiments. For example, pharmaceutical companies may use the method to verify or confirm findings in clinical trials, laboratory tests or medical experiments or may use the method as decision aid, which tests or experiments are to be performed.

Further, documents containing new findings are regularly published. Similarly, data in structured data sources is continuously updated. By means of the described method, it is possible to always use and process the most up-to-date information. Moreover, once an initial graph is created, it can easily be updated on the fly and/or in an incremented way. For example, new information included in newly published documents or added to the structured data sources can be integrated into an initially created graph without the necessity to re-analyze the other documents or to reprocess the whole data basis. The method therefore does not only allow using up-to-date information, but also provides an easily updatable data basis.

Many of the processing steps of the method described herein can also be performed in parallel. Analyzing the plurality of documents in parallel is only one example. Concurrency can be further improved through batch processing and delegation of the analysis to multiple cores of a processor. An increased parallelism does not only speed up the whole data processing, but also allows for good scalability.

Another advantage of the described approach is that it provides the provenance for the information, allowing an end user to access the source (e.g., the document from which the information was extracted) and date of information contained in the graph. This allows human experts to decide whether to take certain information into consideration or not.

In some embodiments, the plurality of documents is analyzed by first receiving or obtaining the plurality of documents from a document data source and then analyzing the plurality of documents by a textual analysis tool to extract three-part propositions from sentences in the plurality of documents. The plurality of documents includes natural language text. The three-part propositions comprise a subject argument as the at least one first entity, an object argument as the at least one second entity, and a relation binding the subject argument with the object argument.

The plurality of documents may comprise domain specific documents written by experts in the respective technical field. For example, the documents may be scientific literature and/or biomedical publications such as books, papers, or articles. Alternatively or additionally, the documents may also comprise medial files (e.g., electronic patient records or digitized documentation). The information included in the documents may be unstructured. For example, the documents comprise completely or partially unstructured text. Unstructured text in the context of the present disclosure may be textual information or natural language text (i.e., free or raw text) in any form. Examples for unstructured text are abstracts or full-texts of publications or text included in electronic health records. The plurality of documents may be stored in a data source of unstructured data (i.e., an unstructured data source) such as in a data repository, in a data base, or as files in a file system. The document data source may be accessible online or may be connected to the Internet or a network.

For the analysis of the plurality of documents, documents relevant may be collected (i.e., harvested) from one or more data sources.

For scientific literature, different data sources already exist. For example, the method may retrieve relevant article abstracts as well as their full-text, when available, from PubMed. PubMed is a bibliographic database of life sciences and biomedical information and comprises more than 30 million citations for biomedical literature from MEDLINE, life science journals, and online books. Most of the citations are accompanied by an abstract. PubMed supports semantic retrieval based on manually reviewed topic annotations using the so-call MeSH vocabulary. The citations are uniformly accessible with PubMedCentral (PMC), which offers the full-text of about 4.7 million articles.

In order to obtain the plurality of documents relevant to a specific use-case from the data source, appropriate semantic queries may be performed. Based on the queries, the method may then retrieve the relevant article citations and extract the title, the abstract text and the topic annotations for each article. In addition, PMC may be queried and any available full-text for the retrieved articles may be retrieved. The inclusion of full-text may improve the prediction results as some important details, information, or associations are only included in the full-text.

The step of retrieving or obtaining the relevant documents from one or more data sources is sometimes also called harvesting or knowledge collection.

After the plurality of documents is retrieved from the data source, the method extracts the knowledge included in the retrieved documents. Despite multiple knowledge acquisition efforts to catalog biological information in a structured form in databases, a considerable amount of knowledge is still buried in natural language text included in the scientific literature. Text mining (i.e., text data mining or text analytics) offers the potential to tap into the knowledge hidden in the ever-increasing body of scientific literature.

Various techniques for identifying and extracting knowledge or information from the plurality of documents may be used. For example, the method may perform a deep syntactic analysis and entity relation extraction to identify subject-verb-object patterns. Alternatively or additionally, natural language processing in single sentences may be applied to find dependency paths (relation types) between entities. Moreover, a relational model may be used that allows an expressive way of capturing statement semantics by modeling n-ary associations between entities.

In a preferred embodiment, a textual analysis tool extracts three-part predictions (i.e., triples), called semantic predications, from sentences in the documents. The predictions comprise a subject argument, an object argument, and a relation that binds them. For example, SemRep developed by the Lister Hill National Center for Biomedical Communications may be used to extract biomedical predications from unstructured text. The result of the knowledge extraction thus may be triples in the form of subject-predicate-object.

In some embodiments, the data or information extracted from the plurality of documents is analyzed and/or categorized to use a uniform terminology or vocabulary.

In order to better integrate the information extracted from the various documents and data sources, a common scheme may be utilized. For example, the Unified Medical Language System (UMLS) is a compendium of many controlled vocabularies in the biomedical sciences. The UMLS was designed and is maintained by the U.S. National Library of Medicine. It provides a mapping structure among these vocabularies and thus allows one to translate among various terminology systems. It may also be viewed as a comprehensive thesaurus and ontology of biomedical concepts.

Two main components of UMLS may be utilized to improve the drug-drug interaction prediction. Metathesaurus forms the base of the UMLS and comprises over 1 million biomedical concepts and 5 million concept names, all of which stem from the over 100 incorporated controlled vocabularies and classification systems. The Metathesaurus therefore may be used to link different names, expressions or terms in the plurality of documents under a single concept. Each concept in the Metathesaurus is assigned one or more semantic types (categories), which are linked with one another through semantic relationships. Further, UMLS provides the Semantic Network which is a catalog of these semantic types and relationships.

If SemRep is used to extract biomedical predications, the subject and object arguments in the predications are concepts from the UMLS and the predicate is one of the semantic relations of the Semantic Network, connecting the semantic types of the subject and object in the context of the specific sentence. Although the documents and the supplementary information come from different sources, authors, or experts, the use of a uniform terminology as provided by UMLS allows creating a common and unified data scheme.

The knowledge extraction process may thus identify associations in the plurality of documents. For example, biomedical entities and relations between the biomedical entities may be extracted from the biomedical literature. These relations may be explicitly defined in the biomedical literature or may only be implicitly disclosed.

The knowledge extraction process may start after all relevant documents are received (i.e., the retrieval of the documents and the knowledge extraction process are performed one after the other) or the knowledge extraction process for one or more documents may already be performed while other documents are still retrieved (e.g., downloaded) from the data source(s) so that the retrieval of the plurality of documents and the knowledge extraction is performed in parallel.

Further, a framework may be created in which different text mining tools are incorporated in a pipeline for extracting the biomedical relations from text, and/or for enriching and categorizing the extracted information.

In some embodiments, the first entity extracted from the plurality of documents represents a drug, a disease, a protein, a chemical, a gene, an organism, a symptom, or a compound. The second entity extracted from the plurality of documents may also represent drug, a disease, a protein, a chemical, a gene, an organism, a symptom or a compound. As discussed above, the extracted information also includes an association between the first entity and the second entity which defines the relationship between the entities.

It follows that the extracted information may include a large variety of relation types. For example, known drug-drug-interactions are extractable from the plurality of documents. In such a case, the first entity represents a first drug. The second entity represents a second drug. In addition, the result of the extraction also specifies that the first entity "interacts" with the second entity. Many other combinations of entities may alternatively or additionally be extracted from the plurality of documents. For example, the information that a certain drug was used to treat a certain disease may be extracted from the plurality of documents as a drug-treats-disease triple. The fact that a certain protein affects a certain gene (i.e., a protein-affects-gene triple) or that a certain gene interacts with another gene (i.e., a gene-gene interaction) may also be extractable from the plurality of documents. Other relation types which may be extracted from the plurality of documents may be that a certain gene belongs to an organism or that a certain disease causes certain symptoms.

The extracted information may also contain information that certain entities do not influence or affect other entities. For example, an analyzed document may disclose that it has been found that a first drug does not interact with a second drug. The extracted information thus may also contain negative statements expressly stating it is known that there is no causal relationship between two entities.

In some embodiments, the supplementary information in the structured data sources comprises general knowledge and/or domain-specific knowledge. The supplementary information may not be included in, may not be extractable from the plurality of documents, or may also be included in the plurality of documents. The structured data source provides access to the supplementary information in a structured and machine-readable format.

The information received from the one or more structured data sources may contain supplementary or additional knowledge. The retrieved and analyzed documents are often written for experts in a respective field and the authors of these documents assume certain knowledge as known. Common general knowledge in a respective field may help to understand relationships or the context of a statement. In addition, special knowledge stored in certain data sources may be taken into account to support a computer system how to interpret certain knowledge.

For example, the supplementary information may comprise information about genes and/or protein interactions. Alternatively or additionally, the structured data sources may provide information about hypernymic relations. Hypernymic relations are also called is_a relations. Hypernymic relations may be retrieved, for example, from biomedical ontologies as available in the Open Biomedical Ontologies format. Biomedical ontologies may be an important source of domain knowledge in the field of biomedicine.

Moreover, an already existing collection of known drug-drug-interactions may be used as supplementary information. For example, the DrugBank database (available at www.drugbank.ca) is a comprehensive, freely accessible, online database containing information on drugs and drug targets. Known drug interactions retrieved from the DrugBank may thus be used as supplementary information.

Structured data in the context of the present disclosure may correspond to data from well-defined databases and/or ontologies (e.g., robust and manually-curated data). The supplementary information in the structured data source(s) may be stored in a standardized or known format or the stored information is annotated (e.g., by human experts) so that the information is machine-readable and machine-understandable by computer systems.

In some embodiments, the plurality of documents and the supplementary information from the structured data source are received from and/or are stored in two or more distinct data sources. Each of the two or more data sources is remote from a data processing system performing the drug-drug interaction estimation.

For example, the method may use information from two different data sources which are located at physically separated places. A first data source stores the plurality of documents. Any data source may be used as first data source as long as it stores and provides access to the documents. A second data source comprises structured data. For example, in an exemplary implementation the second data source may be provided by a file stored in a local file system. Different computer systems or networks may provide access to the information in the different data sources. The different computer systems may be maintained by different organizations. It is therefore obvious that the data sources may not only differ in their content (e.g., structured data and natural language text), but also in how they work, are accessed or how they provide the requested information. The data sources or the content in the data sources may also be provided and maintained by different organizations, institutions or companies.

Furthermore, the method also allows the combination of more than two data sources. For example, the method may use supplementary information from two or more different data sources. Alternatively or additionally, the plurality of documents may be stored in or received from two or more different document repositories.

The advantage of using multiple different data sources is the ability to efficiently combine and analyze data from various sources and thereby identify patterns that can suggest a high probability of a drug-drug interaction. Instead of concentrating only on specific documents and publications (e.g., from a single source), the present disclosure leverages the bigger picture provided by different data types, data formats, and data sources.

In some embodiments, creating the graph comprises adding a node for each first and second entity extracted from the plurality of documents to the graph. A found entity is thus represented by a node in the graph. The nodes in the graph are then connected by edges if an association between the respective entities is derivable from the documents. An edge between two nodes thus represents a known association between the two entities represented by the nodes. The associations are also extracted or derived from the plurality of documents. In addition to the nodes for the entities and the edges for the known associations, supplementary nodes and supplementary edges are added to the graph, based on the supplementary information received from the structured data source(s).

A first type of nodes may thus represent entities found in and extracted from the plurality of documents. For each new entity extracted from a document a new node may be added. For an entity extracted from a document for which a respective node already exists in the graph (e.g., the entity was also mentioned in another earlier analyzed document) there is no need to add a second node for the same entity. After the creation of the graph based on the information extracted from the plurality of documents, there is therefore only one node in the graph for every entity extracted from the plurality of documents. Consequently, the graph includes a single node for each drug, disease, gene, etc..

The nodes in the graph may then be bound if there is a known relation between the entities, for example a drug-drug interaction between two drugs represented by the nodes. In other words, an edge between a first node and a second node may be added to the graph to represent that a first entity represented by the first node is associated with a second entity represented by the second node. In the graph, a specific associated entity pair extracted from a document therefore is represented by two nodes connected by an edge. For example, two nodes representing drugs are connected by an edge if there is a known interaction between these drugs.

As mentioned above, the knowledge extracted from the plurality of documents can be in the form of three-part predications comprised of a subject argument, an object argument, and a relation binding the subject argument with the object argument. This representation allows efficiently creating the graph by adding nodes as defined in the subject argument and the object argument if respective nodes do not already exist in the graph. The relation is then added as edge if not already included in the graph.

The graph with the nodes and edges extracted from the plurality of documents is then further supplemented with the information from the structured data sources. The supplementary information may be added as supplementary nodes. For example, extra nodes for disease, for drugs, for annotations or tags associated with documents may be added. Additionally or alternatively, supplementary information may be added to the graph as supplementary edges. The supplementary edges may store certain additional information (e.g., by labels associated with or attributes stored with the edges or relations).

The information derived from documents and the data received from the structured sources are thus stored in one or more graph structures. The interconnection of all nodes and edges forms the graph.

In some embodiments, the basis or the source for information represented by a certain node is additionally stored in the graph. In particular, the method further adds a plurality of document nodes to the graph. Each document node represents a certain document of the plurality of documents so that only one document node per document is added. A document node of the plurality of document nodes is then connected via a document edge with a node representing an entity, such as a certain drug, if the entity is mentioned in the document represented by the document node.

Thus, in addition to the knowledge extracted from the plurality of documents or the structured data from the data sources, the method may also determine and store from which of the plurality of documents a certain information is extracted. Co-occurrence provides a way of measuring the association between two terms and potentially helps in targeting interesting and clinically important associations. In the present disclosure this type of connection is also called "MENTIONED_IN" relation.

Further, storing the source of information may help to later assess the trustworthiness of certain information or results.

In some embodiments, a subset or all of the plurality of documents are associated with one or more annotations or tags. The associated annotations or tags are also integrated into the graph. Document nodes for the plurality of documents (or a subset thereof) are added to the graph, if respective nodes do not already exist. Similar as for the MENTIONED_IN relations, each document node represents a certain document of the plurality of documents. Nodes for the one or more annotations or tags are then added to the graph if respective nodes do not already exist in the graph so that only one node for each annotation or tag is included in the graph. A document node is connected with a node representing an annotation or a tag if the document represented by the document node is associated with the respective annotation or tag.

The above-described order of creating the document nodes or the nodes for the annotations or tags is only exemplary. The nodes for the annotations or tags may also be created before the document nodes are created or simultaneously (e.g., in parallel processes).

The annotations or tags may categorize a document, index journal articles, add information to a document which is not already included in the document, or summarize the content of a document by catchwords. The annotations or tags may be manually added by domain experts. This may make these edges robust and semantically rich. The annotations or tags may use the above-discussed MeSH vocabulary.

For example, each article in PubMed is associated with a set of MeSH tags and these associations are encoded in the graph in the form of "article-HAS_MESH-concept" triples. These triples express the topic to which each document (e.g., article) is connected and capture knowledge that humans have contributed.

The nodes for annotations and tags thus represent another possible node type in the graph. Similarly, the edges for storing which document is associated with which annotation or tag are another possible edge type in the graph.

The graph thus stores information of different kind and origin in a unified scheme. In particular, known associations between entities are stored in a single data model together with complementary information retrieved from other sources or derived through the analysis and extraction process.

In some embodiments, supervised machine learning techniques are used to identify the at least one new edge.

Supervised machine learning techniques (i.e., supervised machine learning algorithms or supervised machine learning models) are designed to learn by examples. When training a supervised learning algorithm, the training data consists of inputs paired with the correct outputs. During training, the algorithm searches for patterns in the data that correlate with the desired outputs. After training, the supervised learning algorithm takes in new unseen inputs and determines which label the new inputs is classified as based on prior training data. The objective of a supervised learning model is thus to predict a correct label for newly presented input data.

The method may therefore use supervised machine learning techniques in which data samples are generated from different drug pairs found in the graph. The goal is then to find which drugs interact with each other.

Alternatively or additionally, a variety of other machine learning techniques may be applied to the graph in order to predict new edges and properties of nodes or cluster nodes based on connectivity patterns.

In some embodiments, the created graph is analyzed by extracting expressive features for paths between nodes in the created graph. In particular, the method determines paths between a first node and a second node in the created graph. Expressive feature representations for each of the paths are then generated and used as training data for the supervised machine learning techniques (i.e., a classifier).

Data samples for the supervised machine learning algorithms may be generated from different drug pairs found in the graph. The training data may be generated by first aggregating all possible or a subset of the possible paths between drug nodes of a drug pair to be examined. Let $V = v_1, v_2, ..., v_N$ be the set of nodes in the graph (e.g., nodes for UMLS-mapped biomedical concepts), along with the documents in which the concepts are mentioned. Let $E = e_1, e_2, ..., e_M$ denote the set of edges between the nodes. Then, let $d_1$, $d_2$ be the two examined drug nodes and let $\pi^l$ be a path of length $l$ connecting $d_1$ and $d_2$. This path $\pi$ comprises a series of nodes and edges starting from node $d_1$ and ending in node $d_2$ in the form of: $d_1 e_0 v_0 e_1 v_2 e_2 ... e_{l-1} d_2$.

All the possible paths, say $N_{d_1,d_2}$, between the examined drug pair are first determined. Let

$$\pi = \left\{ \pi_1^l, \pi_2^l, ..., \pi_{N_{d_1,d_2}}^l \right\}$$ be the set of all possible paths between the two drugs represented by drug nodes $d_1$ and $d_2$.

After determining all the possible paths, each path is processed in order to represent the path between the nodes by a certain feature representation. For example, the paths in the graph may be represented as frequent relational chains and/or by semantic encoding. Possible interactions between two drugs are then treated as a classification task.

The feature extraction procedure may also incorporate other techniques, such as graph embedding, to capture specific aspects of the nodes and further enhance the results.

Thus, the method may devise expressive feature representations to model known drug-drug interactions based on the graph and use them to predict whether previously unknown drug-drug interactions are probable.

Frequent relation chains (FRC) model paths as a series of relations so that only the relations found in each path are taken into account. Intermediate nodes are ignored. For example, a path with four edges may be represented by $\pi = e_0 e_1 e_2 e_3$. Thus each path is modelled as a series of relations, forming a relational chain. The basic idea behind this representation is that the specific biomedical concepts that are found in the paths are not explicitly interesting, rather the chain of relations that one must transverse in order to get from the starting drug node to the end drug node.

The relation chains may contain both (implicitly) negative and positive drug pairs. The relation chains may be group accordingly. Then, the support for each relational path encountered is calculated. This expresses the frequency of the relational path. An a-priori algorithm with a heuristically optimized support threshold may be used. The most important positive and negative relational chains $f_{rc_1}, f_{rc_2}, ..., f_{rcN}$ may then be generated. For example, let's say that a generated chain for the positive class is the following: IS A - INTERACTS_WITH - IS A. This relational chain expresses the semantic trail: "$d_1$ belongs to some kind of group (IS A) which interacts with (INTERACTS_WITH) another group of substances, that $d_2$ is part of (IS_A)".

After having selected these relational chains, that act as possible indications for positive and negative pairs accordingly, the number of occurrences of each relational chain in the aggregate of paths found between two nodes may be counted. Let all the possible paths between two drugs $d_1$, $d_2$ be

$$\pi_{d_1,d_2} = \left\{ \pi_1^l, \pi_2^l, ..., \pi_{N_{d_1,d_2}}^l \right\}.$$ They are represented with one feature vector

$$x_{d_1,d_2} = \left[ c_{f_{rc_1}}, c_{f_{rc_2}}, ..., c_{f_{rc_N}} \right]^T,$$ containing the occurrence count of each $f_{rci}$ in the collection as follows:

$$c_{fr_{c_1}} = \sum_{j=1}^{N_{d_1,d_2}} \mathbb{1}(rc_j, \pi_j)$$

where

$$\mathbb{1}(rc_i, \pi_j) = 1$$

if $rc_i$ occurs in $\pi_j$ and 0 otherwise. Ultimately, this implies that each drug pair is denoted by the feature vector $\pi_{d_1,d_2}$, where each cell contains the frequency of the corresponding relational chain $f_{rc}$.

As mentioned above, another approach to represent the features is semantic encoding (SE).

Given that a single relational chain of length $l$ between two drugs is $\pi_{d_1,d_2}^l = d_1 e_0 e_1 e_2 \ldots e_{l-1} d_2$ this approach encodes each of the different possible relation types as features. For example, there are 35 different semantic relations $R = \{r_1, r_2, \ldots, r_{35}\}$ in the UMLS Semantic Network. The order of the relations in the chain is also taken into account. One-hot encoding is used for the 35 features on every possible hop. One-hot is a group of bits among which the legal combinations of values are only those with a single high (1) bit and all the others low (0). For example, with $l = 4$ hops the feature vector $f_r$ will have a length of $4 \times 35 = 140$.

As such, for a specific path $\pi_{d_1,d_2}^l$ the corresponding feature vector will be:

$$x_i = \prod_{m=0}^{m=l} [c_{r_1}, c_{r_2}, \ldots, c_{r_{35}}]^T$$

where $c_{r_i}$ denotes the count of the specific relation and $\|$ denotes the concatenation of these occurrence vectors for each hop. For paths $\pi_{d_1,d_2}^m$ with $m < l = 4$ the last $l - m$ elements of the vector are set to zero.

However, as described in connection with feature relation chains, a single feature vector expressing each drug pair is desired. Thus, for each drug pair, the feature vectors of the individual paths leading from $d_1$ to $d_2$ may be aggregated by summing their corresponding feature vectors. Finally, the feature vector for each drug pair will be:

$$x_{d_1,d_2} = \sum_{j=1}^{N_{d_1,d_2}} \prod_{m=0}^{m=l} [c_{r_1}, c_{r_2}, \ldots, c_{r_{35}}]^T$$

with a total length of 140 features.

Moreover, in order to combine the expressiveness of the two extraction methodologies, they may be concatenated. That is, for each drug pair, the concatenation of the corresponding FRC and SE feature vectors is created. The resulting vectors are denoted as FRC + SE.

In some embodiments, machine learning models are generated and trained. The trained machine learning models are then able to classify a drug pair in two classes (i.e., drugs that interact or drugs that do not interact), with a specific confidence score, representing the probability of a drug-drug interaction, based on feature values coming from the paths existing between the two drug nodes in the graph.

The machine learning models may be generated and trained based on the feature representations discussed above. Further, the performance of the models may be measured under an extensive cross-validation scheme.

Specifically, a nested cross-validation scheme may be used to generate robust estimates regarding the performance of the models. An outer 10-fold cv routine is designed to estimate the performance of the model. In each training fold of the outer cv, an inner 5-fold cv scheme may be put in place to tune hyperparameters of the used classifier. The best performing model of each fold may then be used to estimate the performance of the approach. For all the different approaches a random forest may be used as the final predictor. The hyperparameters of each forest (e.g., number of trees, maximum depth, etc.) may be optimized independently for each model. Performance may be calculated using the area under the receiver-operating characteristic (AUROC), while other metrics such as the F1-score and the area under the precision-recall curve (AUPRC) may also be calculated. It is preferred to use measures that take into account the calibration of the classifier because in link prediction problems it is paramount to have an estimate of the robustness of the recommendations.

The above learning procedure may be used to train and test multiple models for the task of predicting drug-drug interactions. However, there are many more steps that could be incorporated (feature selecting, stacking, deep learning approaches, etc.) or those existing could be expanded (more feature engineering, ensemble predictions, error analysis, etc.) to enhance final results and tune the models with regard to the data and the task at hand.

In some embodiments, probable drug-drug interactions are investigated only for a specific disease. In this case, the plurality of documents and/or the supplementary information relate to the specific disease. The interaction between two or more drugs is then estimated for the specific disease by creating a disease-specific graph.

Thus, a decease-specific approach may be applied and an integrated semantic graph from biomedical literature for a specific disease is created.

The specific disease may be received as input by a user (e.g., selected by a user) via a graphical user interface. Alternatively, the disease may be automatically determined by analyzing available information such as patient records. By focusing on a specific disease, along with general medical knowledge the end-user deems relevant, only disease-specific information or data is taken into account. Preferably, all documents in the plurality of documents and received from a respective data source relate to the specific disease. For example, the documents may be annotated as relevant for the specific disease. Documents for other disease may be ignored.

The method may thus also provide an automatic creation, updatability and analysis of a disease-specific graph. That means for each unique disease, a specialized graph is created that contains data focused on this disease.

In some embodiments, the created graph is a knowledge graph.

Knowledge graphs are an established approach for providing insights from data extracted in multiple domains and for aggregating big data in large-scale knowledge bases, which can then be mined in order to identify useful patterns and meta-associations. Based on the nature of the knowledge graph, it is possible to extract meaningful conclusions such as the correlation between chemical compounds and drugs in graphs storing biomedical data.

As described above, knowledge is extracted from the plurality of documents in the form of triples. Knowledge graphs refer to networks that contain specific topic related entities (i.e., nodes) and the related information (i.e., relations or predicate) between entities. The method may therefore use the knowledge graph to store the knowledge extracted from the plurality of documents.

Whichever approach is used to create or construct the knowledge graph, the result can hardly be perfect. Despite the improved accuracy of natural learning processing and data mining techniques, capturing and integrating heterogeneous data under a unified information schema usually results in an incomplete or erroneous knowledge graph. Aiming at increasing the utility of such graphs, the method may further comprise various refinement techniques which add missing knowledge.

It is important to stress that the present methodology may also be used in many different tasks involving different pair of nodes such as drug-diseases, drug-side effects, genes-diseases, etc. This stems from the fact that the whole process - the harvesting, the data extraction, the graph creation, the feature extraction and the resulting predictive model - is use-case agnostic and dependent only on the topological and relational aspects of the paths between different pairs of nodes. As such, the method may also be used for a variety of other prediction tasks, such as link prediction of different relations, node classification, community detection or (sub)-network similarity.

A data processing system or data processing device comprising a processor and a memory is also provided. The processor is configured to perform the method disclosed herein.

A "processor" includes any, hardware system, mechanism or component that processes data, signals or other information. A processor can include a system with a central processing unit, multiple processing units, dedicated circuitry for achieving functionality, or other systems. Processing need not be limited to a geographic location, or have temporal limitations. For example, a processor can perform its functions in real-time, offline, in a batch mode, etc. Portions of processing can be performed at different times and at different locations, by different (or the same) processing systems.

Those skilled in the relevant art will appreciate that the method may be implemented or practiced with other computer system configurations, including without limitation multi-processor systems, network devices, mini-computers, mainframe computers, data processors, and the like. The invention may be embodied in a computer or data processor that is specifically programmed, configured, or constructed to perform the functions described in detail herein. The invention may also be employed in distributed computing environments, where tasks or modules are performed by remote processing devices, which are linked through a communications network such as a LAN, WAN, and/or the Internet. In a distributed computing environment, program modules or subroutines may be located in both local and remote memory storage devices. These program modules or subroutines may, for example, be stored or distributed on computer-readable media, including magnetic and optically readable and removable computer discs, stored as firmware in chips, as well as distributed electronically over the Internet or over other networks (including wireless networks).

Various software/hardware/network architectures may be used. For example, the functions of the described method may be implemented on one computer or shared/distributed among two or more computers in or across a network. Communications between computers implementing embodiments can be accomplished using any electronic, optical, radio frequency signals, or other suitable methods and tools of communication in compliance with known network protocols.

Further, a computer program product is provided. The computer program product comprises instructions which, when the program is executed by a computer or a computing device, cause the computer or the computing device to carry out the above described method.

Any suitable programming language may be used to implement the routines, methods, or programs of the method described herein, including C, C++, Java, JavaScript, HyperText Markup Language (HTML), Python, or any other programming or scripting code.

Further, different programming techniques may be employed such as procedural or object oriented. Any particular routine may execute on a single computer processing device or multiple computer processing devices, a single computer processor or multiple computer processors.

A computer-readable medium comprising the computer program product is also provided. For example, the computer program product may be stored as computer instructions on a non-transitory computer-readable storage medium.

A "computer-readable medium" as used herein may be any medium that can contain, store, communicate, propagate, or transport the program for use by or in connection with an instruction execution system, apparatus, system, or device. The computer-readable medium may be, by way of example only but not by limitation, an electronic, magnetic, optical, electromagnetic, infrared, or semiconductor system, apparatus, system, device, propagation medium, or computer memory. Such computer-readable medium shall be machine readable and include software programming or code that can be human readable (e.g., source code) or machine readable (e.g., object code).

Examples of non-transitory computer-readable media may include random access memories, read-only memories, hard drives, data cartridges, magnetic tapes, floppy diskettes, flash memory drives, optical data storage devices, compact-disc read-only memories, and other appropriate computer memories and data storage devices. Some or all of the software components may reside on a single computer or on any combination of separate computers.

The method discussed herein (or parts thereof) may be implemented in suitable instructions that may reside on the non-transitory computer-readable medium, hardware circuitry or the like, or any combination and that may be translatable by one or more machines.

## BRIEF DESCRIPTION OF THE DRAWINGS

The forgoing and other features and advantages of the invention will become further apparent from the following detailed description read in conjunction with the accompanying drawings. In the drawings, like reference numbers refer to like elements.

Fig. 1 is a schematic drawing illustrating an operating environment for estimating the likelihood that two or more drugs interact with each other in accordance with an embodiment of the invention.

Fig. 2 is a flow diagram illustrating a method according to an embodiment of the invention.

Fig. 3 is a schematic drawing showing a subgraph of a knowledge graph created in an embodiment of the invention.

Fig. 4 is a schematic drawing illustrating paths between two nodes according to an embodiment of the invention.

Fig. 5. is a diagrammatic representation of a data processing system for performing the method disclosed herein.

## DETAILED DESCRIPTION

In the following, embodiments of the invention will be described in detail with reference to the accompanying drawings. It is to be understood that the following description of the embodiments is given only for the purpose of illustration and is not to be taken in a limiting sense. It should be noted that the drawings are to be regarded as being schematic representations only, and elements in the drawings are not necessarily to scale with each other. Rather, the representation of the various elements is chosen such that their function and general purpose become apparent to a person skilled in the art.

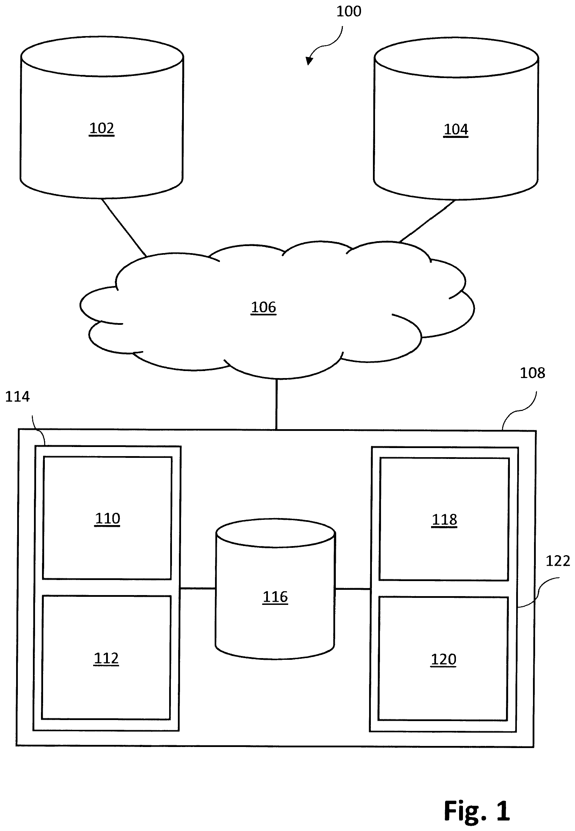

Fig. 1 depicts an illustrative operating environment for estimating the likelihood that two or more drugs interact with each other. A computing environment 100 may include various computer systems, computing devices, networks, and/or other operating infrastructure. In particular, the computing environment 100 includes a drug-drug interaction prediction device 108, a literature data source 102, a database 104 comprising structured data, and a network 106. The literature data source 102 stores a plurality of biomedical publications in the form of natural language text. Each document is associated with annotations. In the database 104 ontologies are stored in a format known and readable by the drug-drug interaction prediction device 108.

The network 106 connects the drug-drug interaction prediction device 108 with the literature data source 102 and the data base 104. The network 106 includes one or more wired networks and/or one or more wireless networks.

The drug-drug interaction prediction device 108 comprises a knowledge graph creation module 114 for creating a knowledge graph. The knowledge graph creation module 114 includes a harvesting module 110 and a knowledge extraction module 112. The harvesting module 110 is configured to receive a plurality of medical publications from the literature data source 102. The received medical publications are then analyzed by the knowledge extraction module 112 to determine biomedical entities and their relations mentioned in the medical publications. The harvesting module 110 is further configured to receive complementary data from the database 104.

Based on the data extracted by the knowledge extraction module 112 and the complementary data from the database 104, a knowledge graph is created by the knowledge graph creation module 114. The knowledge graph creation module 114 then stores the knowledge graph in a knowledge graph management system 116 for further processing. The knowledge graph management system 116 is part of the drug-drug interaction prediction device 108.

Further, a knowledge graph analysis module 122 of the drug-drug interaction prediction device 108 is configured to analyze the knowledge graph stored in the knowledge graph management system 116 to predict probable drug-to-drug interactions. The analysis of the knowledge graph and the prediction of probable interactions are performed by a feature extraction module 118 and an interaction prediction module 120, both included in the drug-drug interaction prediction device 108. In particular, the feature extraction module 118 is configured to determine existing paths included in the knowledge graph stored in the knowledge graph management system 116. The paths in the knowledge graph are then represented by expressive features. The expressive features generated by the feature extraction module 118 are devised by the interaction prediction module 120 to model drug-drug interactions and to predict whether a drug-drug interaction is probable or not.

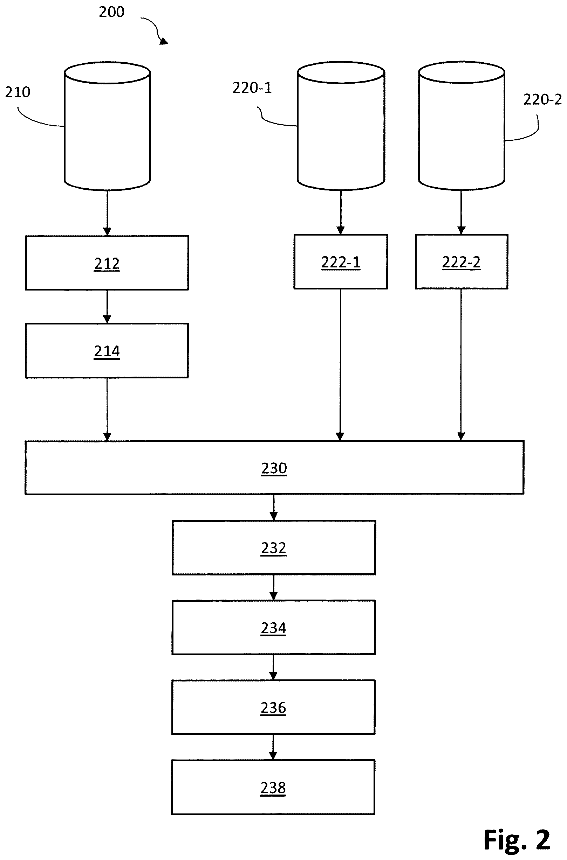

Fig. 2 shows a flow diagram illustrating a process 200 for estimating the likelihood that two or more drugs interact with each other.

A document archive 210 stores a plurality of documents. The plurality of documents represents a collection of biomedical literature. In particular, the documents comprise scientific publications (e.g., articles, papers, books, etc.), which mention known interacting drug pairs and their relations to other biomedical entities. This knowledge is included in the documents as natural language text.

In step 212, a data processing system receives a subset of the documents from the document archive 210 in response to a query from the data processing system to the document archive 210. The query defines a specific disease. All documents received from the document archive 210 relate to the specific disease defined in the query.

In step 214, the documents received from the document archive 210 are analyzed by a textual analysis tool to extract knowledge included in the natural language texts of the documents. The result of the analysis is a plurality of three-part predications (i.e., triples) from sentences in the documents. The three-part predications comprising a subject argument, an object argument, and a relation binding the subject argument with the object argument. The subject and object arguments define two biomedical entities, such as a drug and a disease, while the relation indicates an association between those two, such as a "TREATS" relation.

In addition to the document archive 210, a data source 220-1 comprising biomedical ontologies and a database 220-2 with general knowledge and specific knowledge about the disease is utilized. Data source 220-1 and database 220-2 comprise the data in a standardized or known format so that the data is not only machine-readable, but also machine-understandable by a data processing system.

In step 222-1, the ontologies relevant to the knowledge extracted from the documents are received from data source 220-1. General knowledge and disease-specific knowledge is received from database 220-2 in step 222-2. The ontologies and the knowledge from database 220-2 are hereinafter jointly referred to as supplementary information.

In step 230, data extracted from the documents (i.e., the three-part predications) and the supplementary information is analyzed so that a uniform terminology is used for the information from the document archive 210, the data source 220-1, and the database 220-2.

In step 232, a knowledge graph is created with a unified scheme. The knowledge graph comprises nodes and edges connecting the nodes to store the extracted knowledge and the supplementary information. At least a subset of the edges is associated with attributes and labels to store specific relations between two nodes.

The created knowledge graph is then analyzed by supervised machine learning algorithms to identify new edges in the graph. In step 234, existing paths between two nodes representing drugs to be examined are determined. For each path, an expressive feature representation is then generated. The expressive feature representations are used as training data for the supervised machine learning algorithms.

In step 236, a probable drug-drug interaction is determined based on the identified new edge(s). The probable drug-to-drug interaction is then output in step 238.

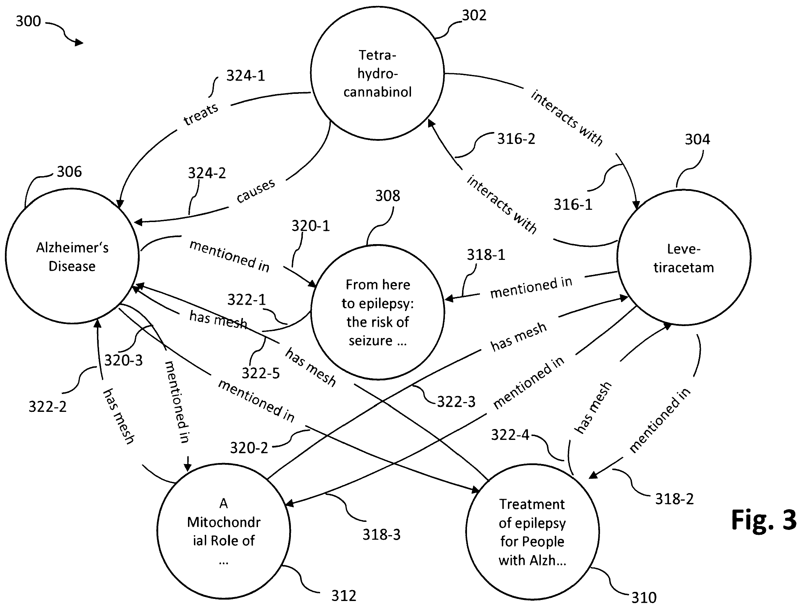

Fig. 3 is a schematic drawing showing an exemplary subgraph 300 of a knowledge graph created in an embodiment of the invention.

The shown subgraph includes six nodes. Two nodes represent drugs. A first drug node 302 is a node for Tetrahydrocannabinol. A second drug node 304 stands for Levetiracetam. The subgraph 300 also comprises a disease node 306 for the Alzheimer's disease. Moreover, three document nodes 308, 310, and 312 are shown. A first document node 308 represents a document with the title "From here to epilepsy: the risk of seizure in patients with Alzheimer's disease" which can be retrieved, for example, from PubMed. A document entitled "Treatment of epilepsy for people with Alzheimer's disease" is illustrated as a second document node 310. Further, a third document node 312 is included in the graph for a document with the title "A mitochondrial role of SV2a protein in aging and Alzheimer's disease: studies with levetiracetam".

Subgraph 300 further shows a number of edges between the nodes. The edges and the information derivable from the edges are discussed in the following paragraphs.

The drug node 302 is connected to the drug node 304 by two edges 316-1 and 316-2. The two edges 316-1 and 316-2 illustrate that it is known that Tetrahydrocannabinol and Levetiracetam interact with each other and therefore are known interacting drug pairs.

It is further shown that Levetiracetam is mentioned in all three documents. In particular, drug node 304 is connected with document node 308 via edge 318-1, is connected with document node 310 via edge 318-2, and is connected with document node 312 via edge 318-3. The subgraph 300 also stores that the Alzheimer's disease is discussed in all three documents. In particular, there are edges 320-1, 320-2, and 320-3 between the disease node 306 and the document nodes 308, 310, and 312.

Furthermore, annotations added to the documents are also part of the subgraph 300. An edge 322-1 between disease node 306 and document node 308 makes clear that the document with the title "From here to epilepsy: the risk of seizure in patients with Alzheimer's disease" is annotated with the disease "Alzheimer's disease". As shown by the subgraph 300, the document represented by the document node 312 is associated with the Alzheimer's disease as represented by edge 322-2 and with Levetiracetam as stored by edge 322-3. The document node 310 is connected via edge 322-4 with the drug node 304 and is connected via 322-5 with the disease node 306 to store that the document represented by the document node 310 is associated with two annotations, namely Levetiracetam and the Alzheimer's disease.

The subgraph 300 also comprises an edge 324-1. The edge 324-1 explains that Tetrahydrocannabinol could be used to treat the Alzheimer's disease. Furthermore, an edge 324-2 shows that Tetrahydrocannabinol also causes the Alzheimer's disease.

The number of nodes, the type of nodes, as well as the number of edges and the type of edges is only exemplary and the described invention is not limited to these numbers or types. In particular, more or less nodes and/or edges may be included in a graph or in a subgraph. Similarly, alternative and additional types of nodes or edges may be used to store the associations extracted from the documents or received from external sources.

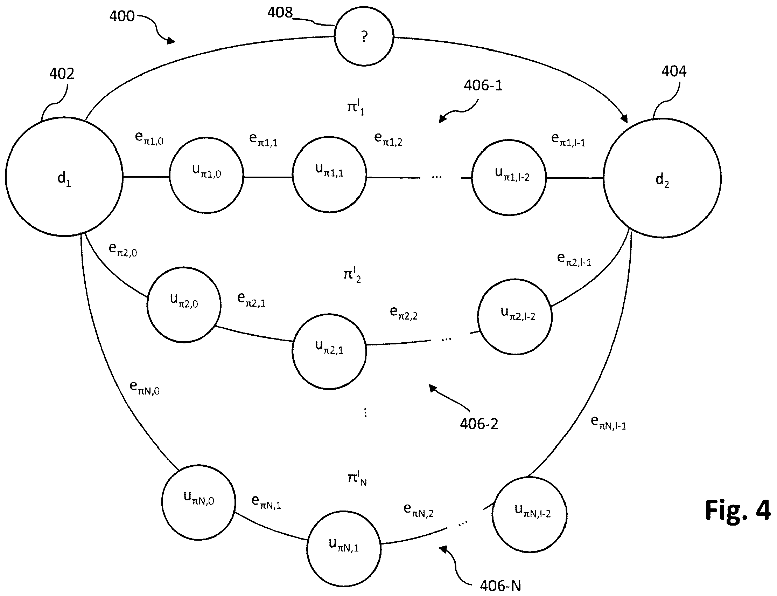

Fig. 4 is a schematic drawing illustrating paths between two nodes according to an embodiment of the invention.

A first drug node 402 represents a first drug with the exemplary name "$d_1$". A second drug node 404 represents a second drug with the exemplary name "$d_2$". Between the first drug node 402 and the second drug node 404, several paths 406-1, 406-2, ..., 406-N exist. Each path 406-1, 406-2, ..., 406-N comprises a plurality of nodes u and edges e. The paths 406-1, 406-2, ..., 406-N are used as training data for a classifier to identify a new edge 408 between the two drug nodes 402 and 404.

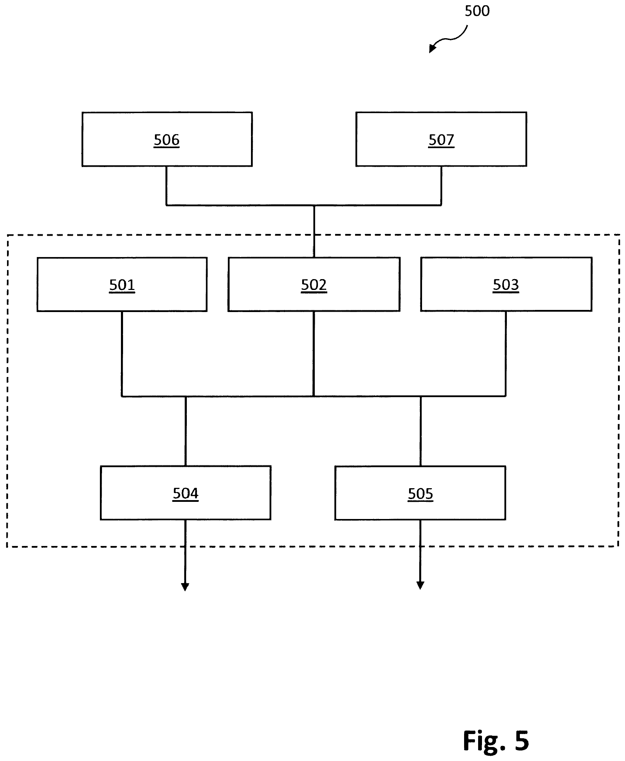

Fig. 5 depicts a diagrammatic representation of a data processing system 500 for implementing a method for estimating a likelihood that two or more drugs interact as described herein.

As shown in Fig. 5, the data processing system 500 includes one or more central processing units (CPU) or processors 501 coupled to one or more user input/output (I/O) devices 502 and memory devices 503. Examples of I/O devices 502 may include, but are not limited to, keyboards, displays, monitors, touch screens, printers, electronic pointing devices such as mice, trackballs, styluses, touch pads, or the like. Examples of memory devices 503 may include, but are not limited to, hard drives (HDs), magnetic disk drives, optical disk drives, magnetic cassettes, tape drives, flash memory cards, random access memories (RAMs), read-only memories (ROMs), smart cards, etc. The data processing system 500 is coupled to a display 506, an information device 507 and various peripheral devices (not shown), such as printers, plotters, speakers, etc. through the I/O devices 502. The data processing system 500 is also coupled to external computers or other devices through a network interface 504, a wireless transceiver 505, or other means that is coupled to a network such as a local area network (LAN), wide area network (WAN), or the Internet.

It will also be appreciated that one or more of the elements depicted in the drawings/figures can also be implemented in a more separated or integrated manner, or even removed or rendered as inoperable in certain cases, as is useful in accordance with a particular application.

As used herein, the terms "comprises", "comprising", "includes", "including", "has", "having", or any other variation thereof, are intended to cover a non-exclusive inclusion. For example, a process, product, article, or apparatus that comprises a list of elements is not necessarily limited only to those elements but may include other elements not expressly listed or inherent to such process, product, article, or apparatus.

Furthermore, the term "or" as used herein is generally intended to mean "and/or" unless otherwise indicated. For example, a condition A or B (as well as a condition A and/or B) is satisfied by any one of the following: A is true (or present) and B is false (or not present), A is false (or not present) and B is true (or present), and both A and B are true (or present).

As used herein, including the claims that follow, a term preceded by "a" or "an" (and "the" when antecedent basis is "a" or "an") includes both singular and plural of such term, unless clearly indicated within the claim otherwise (i.e., that the reference "a" or "an" clearly indicates only the singular or only the plural). Also, as used in the description herein and throughout the claims that follow, the meaning of "in" includes "in" and "on" unless the context clearly dictates otherwise.

While specific embodiments are disclosed herein, various changes and modifications can be made without departing from the scope of the invention. The present embodiments are to be considered in all respects as illustrative and non-restrictive, and all changes coming within the meaning and equivalency range of the appended claims are intended to be embraced therein.

**Claims**

1. A computer-implemented method for estimating a likelihood that two or more drugs interact with each other, the method comprising the steps of:

   analyzing (212, 214) a plurality of documents to extract information, the extracted information comprising at least one first entity associated with at least one second entity;
   receiving (222) supplementary information from a structured data source (104, 220);
   creating (232) a graph (300) to represent the information extracted from the plurality of documents and the supplementary information from the structured data source (104, 220) in a unified scheme, the created graph (300) comprising nodes (304-312) and edges (316-324) connecting the nodes (304-312);
   analyzing (234) the created graph (300) using machine learning techniques to identify, based on paths (406) included in the initially created graph, at least one new edge (408); and
   determining (236) a probable drug-to-drug interaction based on the at least one new edge (408).

2. The method of claim 1, wherein analyzing (212, 214) the plurality of documents comprises:

   receiving (212) or obtaining the plurality of documents from a document data source (102, 210), the plurality of documents including natural language text; and
   analyzing (214) the plurality of documents by a textual analysis tool to extract three-part predications from sentences in the plurality of documents, the three-part predications comprising a subject argument as the at least one first entity, an object argument as the at least one second entity, and a relation binding the subject argument with the object argument.

3. The method of claim 1 or 2, wherein the at least one first entity and/or the at least one second entity includes a drug, a disease, a protein, a chemical, a gene, an organism, a symptom, or a compound; and wherein the at least one first and the at least one second entity are connected by a causal relationship.

4. The method of claim 1, 2, or 3, wherein the supplementary information in the structured data source (104, 220) comprises general knowledge and/or domain-specific knowledge, wherein the supplementary information is not included in or not extractable from the plurality of documents, and wherein the structured data source (104, 220) provides access to the supplementary information in a standardized and/or machine-readable format.

5. The method of any of the preceding claims, wherein the plurality of documents and the supplementary information from the structured data source (104, 220) are received (222) from and are stored in two or more distinct data sources (102, 210; 104, 220), each of the two or more distinct data sources (102, 210; 104, 220) being remote from a data processing system (108) performing the steps of the method.

6. The method of any of the preceding claims, wherein creating (232) the graph (300) comprises:

   adding a node (302, 304, 306) for each of the at least one first entity and the at least one second entity extracted from the plurality of documents to the graph (300); adding the edges (316, 324) between the added nodes (302, 304, 306), wherein an edge (316, 324) between two nodes represents a known association between entities represented by the two nodes (302, 304, 306); and
   adding supplementary nodes and supplementary edges to the graph based on the supplementary information received from the structured data source.

7. The method of claim 6, wherein creating (232) the graph (300) further comprises:

   adding first document nodes (308, 310, 312) to the graph (300), each first document node of the added first document nodes (308, 310, 312) representing a certain document of the plurality of documents; and
   connecting a first document node of the first document nodes (308, 310, 312) via a first document edge (318, 322) with a node (302, 304) for the at least one first entity or the at least one second entity if the node (302, 304, 306) for the at least one first entity or the at least one second entity is extracted from the certain document represented by the first document node (308, 310, 312).

8. The method of claim 6 or 7, wherein at least a subset of the plurality of documents is associated with one or more annotations or tags, and wherein creating (232) the graph (300) further comprises:

   adding second document nodes (308, 310, 312) to the graph (300), each second document node (308, 310, 312) of the added second document nodes (308, 310, 312) representing a certain document of the plurality of documents;

adding nodes (304, 306) for the one or more annotations or tags to the graph (300); and

connecting a second document node of the second document nodes (308, 310, 312) via a second document edge (322) with a node (304, 306) representing an annotations or a tag if the document represented by the node is associated with the respective annotation or tag.

9. The method of any of the preceding claims, wherein supervised machine learning techniques are used to identify the at least one new edge, wherein the graph (300, 400) comprises a first node (402) and a second node (404), and wherein analyzing (234) the created graph (300, 400) to identify at least one new edge (408) comprises:

determining paths (406) between the first node (402) and the second node (404) in the created graph (300, 400);

generating representations for each of the paths (406); and

using the representations as training data for the supervised machine learning techniques.

10. The method of any of the preceding claims, wherein machine learning models are generated and trained, the trained machine learning models providing a score representing the probability of a drug-drug interaction.

11. The method of any of the preceding claims, wherein the plurality of documents and/or the supplementary information relate to a specific disease, and wherein the likelihood that two or more drugs interact with each other are estimated for the specific disease by creating a disease-specific graph (300, 400).

12. The method of any of the preceding claims, wherein the created graph (300, 400) is a knowledge graph.

13. A data processing system comprising a processor (501) configured to perform the method of any of claims 1-12.

14. A computer program product comprising instructions which, when the program is executed by a computer (108, 500), cause the computer (108, 500) to carry out the method of any of claims 1-12.

15. A computer-readable storage medium comprising instructions which, when executed by a computer (108, 500), cause the computer (108, 500) to carry out the method of any of claims 1-12.

Amended claims in accordance with Rule 137(2) EPC.

1. A computer-implemented method for estimating a likelihood that two or more drugs interact with each other when administered together, the method comprising the steps of:

analyzing (212, 214) a plurality of documents to extract information, the extracted information comprising at least one first entity associated with at least one second entity;

receiving (222) supplementary information from a structured data source (104, 220);

creating (232) a graph (300) to represent the information extracted from the plurality of documents and the supplementary information from the structured data source (104, 220) in a unified scheme, the created graph (300) comprising nodes (304-312) and edges (316-324) connecting the nodes (304-312);

analyzing (234) the created graph (300) using machine learning techniques to identify, based on paths (406) included in the initially created graph, at least one new edge (408); and

determining (236) a probable drug-to-drug interaction based on the at least one new edge (408).

2. The method of claim 1, wherein analyzing (212, 214) the plurality of documents comprises:

receiving (212) or obtaining the plurality of documents from a document data source (102, 210), the plurality of documents including natural language text; and

analyzing (214) the plurality of documents by a textual analysis tool to extract three-part predications from sentences in the plurality of documents, the three-part predications comprising a subject argument as the at least one first entity, an object argument as the at least one second entity, and a relation binding the subject argument with the object argument.

3. The method of claim 1 or 2, wherein the at least one first entity and/or the at least one second entity includes a drug, a disease, a protein, a chemical, a gene, an organism, a symptom, or a compound; and wherein the at least one first and the at least one second entity are connected by a causal relationship.

4. The method of claim 1, 2, or 3, wherein the supplementary information in the structured data source (104, 220) comprises general knowledge and/or domain-specific knowledge, wherein the supplementary information is not included in or not extractable from the plurality of documents, and wherein the structured data source (104, 220) provides access to the supplementary information in a standardized and/or machine-readable format.

**5.** The method of any of the preceding claims, wherein the plurality of documents and the supplementary information from the structured data source (104, 220) are received (222) from and are stored in two or more distinct data sources (102, 210; 104, 220), each of the two or more distinct data sources (102, 210; 104, 220) being remote from a data processing system (108) performing the steps of the method.

**6.** The method of any of the preceding claims, wherein creating (232) the graph (300) comprises:

adding a node (302, 304, 306) for each of the at least one first entity and the at least one second entity extracted from the plurality of documents to the graph (300);

adding the edges (316, 324) between the added nodes (302, 304, 306), wherein an edge (316, 324) between two nodes represents a known association between entities represented by the two nodes (302, 304, 306); and

adding supplementary nodes and supplementary edges to the graph based on the supplementary information received from the structured data source.

**7.** The method of claim 6, wherein creating (232) the graph (300) further comprises:

adding first document nodes (308, 310, 312) to the graph (300), each first document node of the added first document nodes (308, 310, 312) representing a certain document of the plurality of documents; and

connecting a first document node of the first document nodes (308, 310, 312) via a first document edge (318, 322) with a node (302, 304) for the at least one first entity or the at least one second entity if the node (302, 304, 306) for the at least one first entity or the at least one second entity is extracted from the certain document represented by the first document node (308, 310, 312).

**8.** The method of claim 6 or 7, wherein at least a subset of the plurality of documents is associated with one or more annotations or tags, and wherein creating (232) the graph (300) further comprises:

adding second document nodes (308, 310, 312) to the graph (300), each second document node (308, 310, 312) of the added second document nodes (308, 310, 312) representing a certain document of the plurality of documents;

adding nodes (304, 306) for the one or more annotations or tags to the graph (300); and

connecting a second document node of the second document nodes (308, 310, 312) via a second document edge (322) with a node (304, 306) representing an annotations or a tag if the document represented by the node is associated with the respective annotation or tag.

**9.** The method of any of the preceding claims, wherein supervised machine learning techniques are used to identify the at least one new edge, wherein the graph (300, 400) comprises a first node (402) and a second node (404), and wherein analyzing (234) the created graph (300, 400) to identify at least one new edge (408) comprises:

determining paths (406) between the first node (402) and the second node (404) in the created graph (300, 400);

generating representations for each of the paths (406); and

using the representations as training data for the supervised machine learning techniques.

**10.** The method of any of the preceding claims, wherein machine learning models are generated and trained, the trained machine learning models providing a score representing the probability of a drug-drug interaction.

**11.** The method of any of the preceding claims, wherein the plurality of documents and/or the supplementary information relate to a specific disease, and wherein the likelihood that two or more drugs interact with each other are estimated for the specific disease by creating a disease-specific graph (300, 400).

**12.** The method of any of the preceding claims, wherein the created graph (300, 400) is a knowledge graph.

**13.** A data processing system comprising a processor (501) configured to perform the method of any of claims 1-12.

**14.** A computer program product comprising instructions which, when the program is executed by a computer (108, 500), cause the computer (108, 500) to carry out the method of any of claims 1-12.

**15.** A computer-readable storage medium comprising instructions which, when executed by a computer (108, 500), cause the computer (108, 500) to carry out the method of any of claims 1-12.

**1.** A computer-implemented method for estimating a likelihood that two or more drugs interact with each other when administered together, the method comprising the steps of:

analyzing (212, 214) a plurality of documents to extract information, the extracted information comprising at least one first entity associated with at least one second entity;

receiving (222) supplementary information from a structured data source (104, 220);

creating (232) a graph (300) to represent the information extracted from the plurality of documents and the supplementary information from the structured data source (104, 220) in a unified scheme, the created graph (300) comprising nodes (304-312) and edges (316-324) connecting the nodes (304-312);

analyzing (234) the created graph (300) using supervised machine learning techniques to identify, based on paths (406) included in the initially created graph, at least one new edge (408); and

determining (236) a probable drug-to-drug interaction based on the at least one new edge (408); wherein the at least one first entity and/or the at least one second entity includes a drug, a disease, a protein, a chemical, a gene, an organism, a symptom, or a compound, and wherein the at least one first and the at least one second entity are connected by a causal relationship; and

wherein the created graph (300, 400) comprises a first node (402) and a second node (404), and wherein analyzing (234) the created graph (300, 400) to identify at least one new edge (408) comprises:

determining paths (406) between the first node (402) and the second node (404) in the created graph (300, 400);

generating representations for each of the paths (406); and

using the representations as training data for the supervised machine learning techniques.

2. The method of claim 1, wherein analyzing (212, 214) the plurality of documents comprises:

receiving (212) or obtaining the plurality of documents from a document data source (102, 210), the plurality of documents including natural language text; and

analyzing (214) the plurality of documents by a textual analysis tool to extract three-part predications from sentences in the plurality of documents, the three-part predications comprising a subject argument as the at least one first entity, an object argument as the at least one second entity, and a relation binding the subject argument with the object argument.

3. The method of claim 1 or 2, wherein the supplementary information in the structured data source (104, 220) comprises general knowledge and/or domain-specific knowledge, wherein the supplementary information is not included in or not extractable from the plurality of documents, and wherein the structured data source (104, 220) provides access to the supplementary information in a standardized and/or machine-readable format.

4. The method of any of the preceding claims, wherein the plurality of documents and the supplementary information from the structured data source (104, 220) are received (222) from and are stored in two or more distinct data sources (102, 210; 104, 220), each of the two or more distinct data sources (102, 210; 104, 220) being remote from a data processing system (108) performing the steps of the method.

5. The method of any of the preceding claims, wherein creating (232) the graph (300) comprises:

adding a node (302, 304, 306) for each of the at least one first entity and the at least one second entity extracted from the plurality of documents to the graph (300);

adding the edges (316, 324) between the added nodes (302, 304, 306), wherein an edge (316, 324) between two nodes represents a known association between entities represented by the two nodes (302, 304, 306); and

adding supplementary nodes and supplementary edges to the graph based on the supplementary information received from the structured data source.

6. The method of claim 5, wherein creating (232) the graph (300) further comprises:

adding first document nodes (308, 310, 312) to the graph (300), each first document node of the added first document nodes (308, 310, 312) representing a certain document of the plurality of documents; and

connecting a first document node of the first document nodes (308, 310, 312) via a first document edge (318, 322) with a node (302, 304) for the at least one first entity or the at least one second entity if the node (302, 304, 306) for the at least one first entity or the at least one second entity is extracted from the certain document represented by the first document node (308, 310, 312).

7. The method of claim 5 or 6, wherein at least a subset of the plurality of documents is associated with one or more annotations or tags, and wherein creating (232) the graph (300) further comprises:

> adding second document nodes (308, 310, 312) to the graph (300), each second document node (308, 310, 312) of the added second document nodes (308, 310, 312) representing a certain document of the plurality of documents;
> adding nodes (304, 306) for the one or more annotations or tags to the graph (300); and connecting a second document node of the second document nodes (308, 310, 312) via a second document edge (322) with a node (304, 306) representing an annotations or a tag if the document represented by the node is associated with the respective annotation or tag.

**8.** The method of any of the preceding claims, wherein machine learning models are generated and trained, the trained machine learning models providing a score representing the probability of a drug-drug interaction.

**9.** The method of any of the preceding claims, wherein the plurality of documents and/or the supplementary information relate to a specific disease, and wherein the likelihood that two or more drugs interact with each other are estimated for the specific disease by creating a disease-specific graph (300, 400).

**10.** The method of any of the preceding claims, wherein the created graph (300, 400) is a knowledge graph.

**11.** A data processing system comprising a processor (501) configured to perform the method of any of claims 1-10.

**12.** A computer program product comprising instructions which, when the program is executed by a computer (108, 500), cause the computer (108, 500) to carry out the method of any of claims 1-10.

**13.** A computer-readable storage medium comprising instructions which, when executed by a computer (108, 500), cause the computer (108, 500) to carry out the method of any of claims 1-10.

**Fig. 1**

**Fig. 2**

Fig. 3

Fig. 4

500

| | |
|---|---|
| 506 | 507 |

| | | |
|---|---|---|
| 501 | 502 | 503 |

| | |
|---|---|
| 504 | 505 |

**Fig. 5**

Europäisches
Patentamt

European
Patent Office

Office européen
des brevets

**EUROPEAN SEARCH REPORT**

Application Number

EP 20 38 6008

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/098993 A1 (MAS BENAVENTE JOSE MANUEL [ES] ET AL) 28 April 2011 (2011-04-28) * paragraphs [0038], [0071] - [0078], [0088], [0118], [0167], [0179], [0223], [0237]; figure 1 * ----- | 1-15 | INV. G16H50/50 G16H70/40 |

TECHNICAL FIELDS
SEARCHED (IPC)

G16H

The present search report has been drawn up for all claims

| Place of search | Date of completion of the search | Examiner |
|---|---|---|
| Munich | 18 June 2020 | Samulowitz, Michael |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 38 6008

This annex lists the patent family members relating to the patent documents cited in the above-mentioned European search report.
The members are as contained in the European Patent Office EDP file on
The European Patent Office is in no way liable for these particulars which are merely given for the purpose of information.

18-06-2020

| Patent document cited in search report | Publication date | Patent family member(s) | Publication date |
|---|---|---|---|
| US 2011098993 A1 | 28-04-2011 | US 2011098993 A1<br>WO 2011051805 A1 | 28-04-2011<br>05-05-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6370478 B